# EUROPEAN PATENT SPECIFICATION

(11) **EP 1 192 273 B1**
(45) Date of publication and mention of the grant of the patent: **28.01.2004**
(21) Application number: 00919078.6
(22) Date of filing: 19.04.2000
(51) Int. Cl.: C12Q 1/68, G01N 33/53

(54) **METHOD FOR DETECTING MISMATCHES OR MUTATIONS IN NUCLEIC ACID**
VERFAHREN ZUM NACHWEIS VON FEHLPAARUNGEN ODER MUTATIONEN IN NUKLEINSÄUREN
TECHNIQUE PERMETTANT DE DETECTER ET DE MODIFIER DES MESAPPARIEMENTS ET DES MUTATIONS DANS L'ACIDE NUCLEIQUE

(30) Priority: 19.04.1999 JP 11091499
(43) Date of publication of application: 03.04.2002
(73) Proprietor: Amersham Biosciences KK, Shinjuku-ku, Tokyo (JP)
(72) Inventor: GOTOH, Masanori, Tokyo146-0095 (JP); WHITTIER, Robert F., Ibaraki 300-0832 (JP)
(74) Representative: Rollins, Anthony John
(86) International application number: PCT/IB2000/000489
(87) International publication number: WO 2000/063691

(56) References cited:
- WO-A-93/22457
- WO-A-95/12689
- WO-A-96/23895
- WO-A-97/33903
- WO-A-99/06591
- WO-A-99/58717
- US-A- 5 459 039
- US-A- 5 556 750
- ZHARKOV DMITRY O ET AL: "NH-2-terminal proline acts as a nucleophile in the glycosylase/AP-lyase reaction catalyzed by Escherichia coli formamidopyrimidine-DNA glycosylase (Fpg) protein." JOURNAL OF BIOLOGICAL CHEMISTRY, vol. 272, no. 8, 1997, pages 5335-5341, XP002180727 ISSN: 0021-9258
- KARAKAYA ASUMAN ET AL: "Kinetics of excision of purine lesions from DNA by Escherichia coli Fpg protein." NUCLEIC ACIDS RESEARCH, vol. 25, no. 3, 1997, pages 474-479, XP002180728 ISSN: 0305-1048
- DATABASE BIOSIS [Online] BIOSCIENCES INFORMATION SERVICE, PHILADELPHIA, PA, US; 1992 MICHAELS M L ET AL: "EVIDENCE THAT MUTY AND MUTM COMBINE TO PREVENT MUTATIONS BY AN OXIDATIVELY DAMAGED FROM OF GUANINE IN DNA" Database accession no. PREV199294119589 XP002180729 & PROCEEDINGS OF THE NATIONAL ACADEMY OF SCIENCES OF THE UNITED STATES, vol. 89, no. 15, 1992, pages 7022-7025, 1992 ISSN: 0027-8424

## Description

### Field of the Invention

The present invention relates to a method for detecting mismatches in a double-stranded nucleic acid, a method for detecting a nucleic acid with mutations, and a method for separating a double-stranded nucleic acid with mismatches, in which methods MutM protein is used.

### Background Art

*E. coli* MutS is a protein that recognizes and binds to mismatches (S-S Su et al., J. Biol. Chem., 263, 6829-6835 (1988)). Recently, a method for detecting mismatches and a gene diagnosis method using MutS have been developed (M. Gotoh et al., Genet. Anal., 14, 47-50 (1997)). The strength of MutS binding is known to vary in the kind of base mismatch. In particular, the binding of MutS to pyrimidine-pyrimidine mismatches is weak (M. Gotoh et al., Genet. Anal., 14, 47-50 (1997)). Therefore, it is very likely that mutations would be missed if MutS is applied for gene diagnosis. On the other hand, *E. coli* MutM is a protein that inhibits transversion mutation from guanine/cytosine to thymine/adenine (M. Cabrera et al., J. Bacteriol., 170, 5405-5407 (1988)). *E. coli* MutM recognizes and removes the oxidized base 8-oxoguanine in the context of an 8-oxoguanine/cytosine base pair to suppress transversion mutation (M. L. Michaels et al., Biochemistry, 31, 10964-10968 (1992)).

With respect to cleavage at mismatched base pairs, celery nuclease CEL1 (C. A. Oleylowski et al., Nucleic Acids Res., 26, 4597-4602 (1998)) and T4 phage endonuclease VII (R. Youil et al., Proc. Natl. Acad. Sci. USA, 92, 89-91 (1995)) are known to cleave mismatches. Discrimination among different kinds of base pair mismatches is rather low for these enzymes, because they cleave almost all mismatches.

### Problems to Be Solved by the Invention

Objectives of the present invention are to provide (1) a method for effectively detecting mismatches or mutations in a nucleic acid, (2) a method for effectively separating a population of nucleic acid molecules based upon the presence or absence of mismatches and (3) a method for effectively modifying a mismatch-bearing nucleic acid, using MutM protein in all three methods. Particularly, the method enables effective detection of mismatches between pyrimidines, which has been difficult to achieve by conventional methods using MutS protein. The present invention also provides a method for separating nucleic acids based upon the presence or absence of mismatches and a method for modifying a nucleic acid with such mismatches.

In one embodiment, the present invention provides a gene diagnosis method using the ability of MutM protein to recognize mismatches, a method for purifying a DNA amplification product and a method for labeling a mutant nucleic acid.

### Means of Solving the Problems

The present inventors have tested the ability of *E. coli* MutM to recognize mismatches in a DNA and found that MutM protein can recognize all kinds of mismatches involving cytosine (Figure 1). Moreover, the present inventors have found that MutM protein also binds to not only a single mismatch but also to multiple continuous mismatches (Figure 2). In addition, the present inventors have found that MutM protein also binds to not only single base-single base mismatches but also to single base-multiple base mismatches, in which multiple bases are inserted into one strand of a double-stranded nucleic acid (Figure 3). Moreover, the present inventors have found that MutM protein also binds to mismatches generated by deletion or insertion of a single or multiple bases in one strand of a double-stranded nucleic acid (Figure 4).

Furthermore, the present inventors have succeeded in separating a DNA with and without mismatches from polymerase chain reaction products using *E. coli* MutM. In other words, the present inventors have found that using MutM protein enables detecting mismatches in a DNA and separating a DNA with mismatches.

Furthermore, the present inventors have found that such ability of MutM protein is applicable to gene diagnoses.

Furthermore, the present inventors have succeeded in cleaving a double-stranded nucleic acid at mismatches. In other words, the present inventors have found that using MutM protein, enables separate a double-stranded nucleic acid from a double-stranded nucleic acid sample and modify a double-stranded nucleic acid at mismatches.

Thus, the present invention relates to a method for detecting mismatches in a double-stranded nucleic acid or a nucleic acid with mutations using MutM protein, and a method for separating a double-stranded nucleic acid with mismatches or a nucleic acid with mutations using MutM protein. More specifically, the present invention relates to:
(1) a method for detecting mismatches in a double-stranded nucleic acid, wherein the method comprises
   (a) contacting a test double-stranded nucleic acid with MutM protein and
   (b) detecting the binding of said double-stranded nucleic acid to said protein;
(2) the method of (1), wherein MutM protein is derived from *E. coli*;
(3) the method of (1) or (2), wherein the test double-stranded nucleic acid is binding to a support or labeled so as to bind to a support;
(4) the method of (1) or (2), wherein MutM protein is binding to a support or labeled so as to bind to a support;
(5) the method of any one of (1), (2), or (4), wherein the test double-stranded nucleic acid is detectably labeled;
(6) the method of any one of (1) to (3), wherein MutM protein is detectably labeled;
(7) a method for detecting mutations in a nucleic acid, wherein the method comprises
   (a) providing a test nucleic acid and a control nucleic acid,
   (b) hybridizing said test nucleic acid with the control nucleic acid,
   (c) contacting the double-stranded nucleic acid formed by hybridization with MutM protein, and
   (d) detecting the complex between heteroduplex nucleic acid in said double-stranded nucleic acid and said protein;
(8) the method of (7), wherein MutM protein is derived from *E. coli*;
(9) the method of (7) or (8), wherein the test nucleic acid or the control nucleic acid is binding to a support or labeled so as to bind to a support;
(10) the method of (7) or (8), wherein MutM protein is binding to a support or labeled so as to bind to a support;
(11) the method of any one of (7), (8), or (10), wherein the test nucleic acid or the control nucleic acid is detectably labeled;
(12) the method of any one of (7) to (9), wherein MutM protein is detectably labeled;
(13) a method for separating a double-stranded nucleic acid with mismatches from a double-stranded nucleic acid sample, wherein the method comprises
   (a) contacting the double-stranded nucleic acid sample with MutM protein and
   (b) collecting a double-stranded nucleic acid that forms a complex with MutM protein from the double-stranded nucleic acid sample;
(14) a method for separating a double-stranded nucleic acid without mismatches from a double-stranded nucleic acid sample, wherein the method comprises
   (a) contacting the double-stranded nucleic acid sample with MutM protein and
   (b) collecting a double-stranded nucleic acid that does not bind to MutM protein from the double-stranded nucleic acid sample;
(15) the method of (13) or (14), wherein MutM protein is derived from *E. coli*;
(16) the method of any one of (13) to (15), wherein the double-stranded nucleic acid is a DNA amplification product; and
(17) the method of any one of (13) to (16), wherein MutM protein is binding to a support or labeled so as to bind to a support.

"Mismatch"used herein means that a base pair selected from adenine (A), guanine (G), cytosine (C), and thymine (T) (or uracil (U) in RNA) is not a normal base pair (A/T or G/C). In this invention, a "mismatch" includes not only a single mismatch but also multiple, continuous mismatches that are generated by inserting or deleting a single or multiple bases or their combinations.

"Mutation" used herein means a base (or a base pair in the case of a double-stranded nucleic acid) in a sample nucleic acid different from that in a control nucleic acid. Thus polymorphisms (specific sequence variants occurring at greater than 1% frequency within natural populations) are included in this definition of "mutation".

"A nucleic acid" used herein includes a DNA or an RNA, for example, a cDNA, a genomic DNA, an mRNA, or a synthetic oligonucleotide. It also includes a single-stranded and a double-stranded nucleic acid, and a linear and a circular nucleic acid.

"A control nucleic acid" means a reference nucleic acid without mutations. In the case that natural polymorphisms exist in the base sequence, one of the most common sequence variants is chosen as the reference. "A sample nucleic acid" means a nucleic acid suspected to have bases different from those in the control nucleic acid (mutations). A sample nucleic acid is the same as a control nucleic acid if it does not have mutations. In other words, it is different from a control nucleic acid only at the mutation sites if it has mutations. For example, when mutations in genes of a patient suspected to have a genetic disease are to be detected, a gene of the patient suspected to have mutations is a sample nucleic acid and the corresponding gene of a healthy subject is a control nucleic acid.

"A heteroduplex nucleic acid" means a substantially complementary double-stranded nucleic acid but with regions that are not complementary due to one or multiple mismatches. "A test nucleic acid" is formed by melting and re-annealing control and sample nucleic acids together so that double-stranded nucleic acid molecules form which are composed one strand each from the control and sample nucleic acids. Thus if a sample nucleic acid contains mutations, a test nucleic acid derived from it will include heteroduplex molecules.

### Mode for Implementing the Invention

In one aspect, the present invention relates to a method for detecting mismatches in a double-stranded nucleic acid using MutM protein. The method of the present invention utilizes the ability of MutM protein to recognize mismatches in a double-stranded nucleic acid. In this method, mismatches are detected in terms of binding of MutM protein to a test double-stranded nucleic acid. Therefore, the method of the present invention comprises (a) contacting a test double-stranded nucleic acid with MutM protein and (b) detecting the binding of said double-stranded nucleic acid to said protein.

The method of the present invention is particularly suitable for detecting mismatch base pairs involving cytosine (C/A, C/T, C/C). The method can also be applied to detecting multiple continuous mismatches, mismatches between a single base and multiple bases, and mismatches generated by deleting and/or inserting a single or multiple bases in at least one strand of a double-stranded nucleic acid. In particular, the method is suitable for detecting mismatches with cytosine.

MutM protein derived from *E. coli* is preferable as the ''MutM protein" used for the method of the present invention. However, there is no limitation on its source as far as it can recognize mismatches in a double-stranded nucleic acid. To date, yeast Ogg1 and Ogg2 (P. A. van der Kemp et al., Proc. Natl. Acad. Sci. USA, 93, 5197-5202 (1996)), mouse Ogg1 (T. A. Rosenquist et al., Proc. Natl. Acad. Sci. USA, 94, 7429-7434 (1997)), *Thermus thermophilus* MutM (T. Mikawa et al., Nucleic Acids Res., 26, 903-910 (1998)), *Arabidopsis thaliana* AtMMH1 and AtMMH2 (T. Ohtsubo et al., Mol. Gen. Genet., 259, 577-590 (1998)), human Ogg1 (K. Arai et al., Oncogene, 14, 2857-2861 (1997)), etc. are known as homologues to MutM proteins as well as to that of *E*.*coli*. The partial peptides of these proteins can also be used as long as they can recognize mismatches in a double-stranded nucleic acid.

Moreover, proteins (mutants) generated by performing one or more amino acid substitutions, deletions, additions, and/or insertions into the wild-type proteins can be used as long as they can recognize mismatches in a double-stranded nucleic acid. Such mutants can occur spontaneously or can be prepared artificially. Many methods for introducing amino acid mutations into proteins are well known. For example, the known methods include site-directed mutagenesis (W. P. Deng and J. A. Nickoloff, Anal. Biochem., 200, 81 (1992), and K. L. Makamaya and F. Eckstein, Nucleic Acids Res., 14, 9679-9698 (1986)) and random mutagenesis such as the method using *E. coli* XL1-Red strain (Stratagene), which is deficient in basic repair systems, and the method in which bases are modified chemically using sodium nitrite (J.-J. Diaz et al., BioTechnique, 11, 204-211 (1991)).

MutM protein can be a fusion protein with another protein such as glutathione-S-transferase.

MutM protein can also be prepared as a wild-type or recombinant protein by a known method in which anion exchange column chromatography, cation exchange column chromatography, gel filtration column chromatography, ammonium sulfate fractionation, etc. are combined (S. Boiteux et al., EMBO J., 6, 3177-3183 (1987)). The recombinant protein can be easily prepared by chromatography alone using a cation exchange column and gel filtration column if the expression level is high.

The double-stranded nucleic acid used in the present invention can be the double-stranded nucleic acid to be examined to determine whether or not it has mutations. The double-stranded nucleic acid can be in any form of a double-stranded DNA, double-stranded RNA, or DNA/RNA. The double-stranded nucleic acid can be used in the test as it is or after being amplified in a vector such as a phage or plasmid. A double-stranded nucleic acid amplified by polymerase chain reaction (PCR) can also be used.

In the present invention, the test double-stranded nucleic acid can be contacted with MutM protein under conditions (appropriate pH, solvent, ionic environment, and temperature) in which said protein can bind to the mismatch region(s) of the test double-stranded nucleic acid. An example of the buffer composition is 50 mM Hepes-KOH (pH 7.2), 100 mM KCl, 1 mM EDTA, and 1mM DTT. An example of temperature is 25°C and that of the reaction time is about 1 to 10 minutes. The conditions mentioned above are only examples; more precise conditions of reaction temperature, salt concentration, the kind of ions, and buffer pH can be appropriately selected.

When single-stranded nucleic acids are expected to remain during the preparation of double-stranded nucleic acids, the single-stranded nucleic acids should be removed by, for example, using a MicroSpin S-300 HR column (Amersham Pharmacia Biotech) or blocking the nucleic acids with *E. coli* SSB protein beforehand.

The method for detecting the binding of a test double-stranded nucleic acid to MutM protein is not limited. Examples of the detection system are as follows.
(1) A test double-stranded nucleic acid is immobilized on a support or labeled so as to be immobilized on a support, and MutM protein is used without being labeled. The nucleic acid can be labeled so as to be immobilized to a support by binding one of the two substances with affinity to the nucleic acid and the other to the support. Biotin-avidin system and antibody-antigen system (for example, anti-digoxigenin antibody and digoxigenin) may be used as these substances. MutM protein can be directly detected using a detection system with a sensor using, for example, a quartz oscillator, surface plasmon resonance, or porous silicon. Examples of the support include the gold on a quartz oscillator, the gold on the sensing element of a surface plasmon sensor, or the silicon of a porous silicon sensor. Substances can be immobilized on the support directly or through a matrix of dextran and so on (K. Bondeson et al., FEBS Letter, 423, 307-313 (1993)).
(2) A test double-stranded nucleic acid is immobilized on or labeled so as to be immobilized on a support. After MutM protein is reacted with the test double-stranded nucleic acid, the MutM protein unbound from the nucleic acid is removed, then the remaining MutM protein is detected. The nucleic acid can be labeled with biotin or compounds recognized by antibodies so as to be immobilized on a support. MutM protein can also be labeled with a detectable compound including radioactive substances such as ³⁵S or ³H, a fluorescent substance such as FITC, biotin, or a compound detectable with an antibody such as FITC. Alternatively, MutM protein can be detected without labeling if an antibody against MutM protein is used. The complex between an antibody and MutM protein can be detected by binding another antibody to the complex. An antibody to which alkaline phosphatase, horseradish peroxidase (HRP), or β-galactosidase binds can be used. In this case, the detection can be performed by a known coloring method or chemiluminescence method in which the activity of alkaline phosphatase, horseradish peroxidase (HRP), or β-galactosidase is used. The protein labeled with a radioactive or fluorescence substance can be directly detected. Any support that separates solid from liquid, such as membrane filters, microtiter plates, chromatographic carriers, or magnetic beads, can be used. When biotin is to be detected, avidin or streptavidin is immobilized on the support. When a compound recognizable by an antibody is to be detected, an antibody is immobilized on a support. Antibodies can be immobilized by physical adsorption or chemical binding using a crosslinking agent directly or through protein A or protein G.
(3) A test double-stranded nucleic acid is detectably labeled and MutM protein is immobilized on or labeled so as to be immobilized on a support. The nucleic acid can be labeled with a radioactive substance such as ³⁵S or ³H, a fluorescent substance such as FITC or Cy5, an enzyme such as HRP, or a compound such as biotin, detectable with avidin or streptavidin, or FITC or digoxigenin, detectable with an antibody. MutM protein can be immobilized on a support directly by physical adsorption or chemical binding using a crosslinking agent. Alternatively, the protein can be labeled with biotin or a compound detectable with an antibody (antigen) and immobilized on a support through avidin or streptavidin for biotin or through an antibody for the antigen. Furthermore, the N-terminus or C-terminus of MutM protein is tagged with a short histidine residue and immobilized through metal by utilizing the chelate action of the tag. The detection system and the support used are the same as those mentioned in (2).
(4) A test double-stranded nucleic acid is detectably labeled without labeling the MutM protein. The solid and liquid are separated, such as by immunoprecipitation, using anti-MutM antibody. The antibody against MutM protein can be immobilized through protein A or protein G bound to a support such as beads. A nucleic acid can be labeled as described in (3). The detection system and the support used are the same as those mentioned in (2).
(5) A test double-stranded nucleic acid is not labeled, and MutM protein is immobilized on, or labeled so as to be immobilized on, a support. The labeling substance that can be immobilized on a support is the same as described in (3). A double-stranded nucleic acid can be directly detected in the same manner as in (1), for example, with a sensor using a quartz oscillator, surface plasmon resonance, or porous silicon. The support is the same as described in (1). The protein can be immobilized on the sensing element of a surface plasmon resonance sensor by the method of I. Chaiken (Anal. Biochem., 201, 197-210 (1992)).

If significant binding of the test double-stranded nucleic acid to MutM proteins is detected, it is judged that the test double-stranded nucleic acid has mismatches. In contrast, if significant binding of the double-stranded nucleic acid to MutM proteins is not detected, it is judged that the test double-stranded nucleic acid does not have mismatches.

The method of the present invention for detecting mismatches includes quantification of mismatches. For example, it is possible to determine the amount of mismatches in a nucleic acid sample by measuring the amount of MutM protein bound to a nucleic acid with mismatches using a labeled protein or an antibody. It is also possible to determine the proportion of mismatched DNA in a sample by labeling the whole nucleic acid sample, binding MutM protein to the sample, and measuring the proportion of the nucleic acid that has formed a complex to the whole nucleic acid sample.

In another aspect, the present invention relates to a method for detecting mutations in a nucleic acid using the MutM protein. In one embodiment, this detection method can be used to examine whether or not a specific gene of a patient suspected to have genetic disease has mutations by examining whether or not the gene derived from the patient and the gene of a healthy subject have the same nucleotide sequence. The method of the present invention enables detecting mutations wherever they exist in a test gene. The method is also superior in that the mutation site of a test gene or the kind of mutation need not to be known.

The principle of this detection method is as follows. A test nucleic acid suspected to have mutations and a control nucleic acid (a nucleic acid without mutations) are prepared and hybridized with each other. If the test nucleic acid has mutations, a heteroduplex nucleic acid (a nucleic acid with mismatch(es)) is generated by hybridizing with the control nucleic acid. In contrast, if the test nucleic acid does not have mutations, only a homoduplex nucleic acid is generated, and no heteroduplex nucleic acid is generated. If the MutM protein is contacted with the double-stranded nucleic acid formed by hybridization, the MutM protein binds to the heteroduplex nucleic acid, which has mismatches, but does not bind to the homoduplex nucleic acid. Therefore, it is possible to judge whether or not a test nucleic acid has mutations by detecting the binding of the MutM protein to the double-stranded nucleic acid.

More specifically, the detection method of the present invention comprises
(a) providing a test nucleic acid and a control nucleic acid,
(b) hybridizing the test nucleic acid with the control nucleic acid,
(c) contacting the double-stranded nucleic acid formed by hybridization with an MutM protein, and
(d) detecting the complex between the heteroduplex nucleic acid in the double-stranded nucleic acid and the protein.

There is no limitation on the test nucleic acid used. Any desired nucleic acid to be examined to determine if it has mutations can be used. A control nucleic acid corresponds to a test nucleic acid. In other words, if the test nucleic acid does not have mutations, it is the same nucleic acid as the control nucleic acid. The word "the same" means that both are the same within the region where they hybridize with each other. The length can be different but should be the same if possible. A test nucleic acid and a control nucleic acid can be either single-stranded or double-stranded. When they are single-stranded, they are complementary to each other provided that the test nucleic acid does not have mutations.

In the method of the present invention, a test nucleic acid and a control nucleic acid are hybridized. (If they are double-stranded, both are denatured and dissociated to form single-stranded nucleic acids and the single-stranded nucleic acids are hybridized.) By doing so, double-stranded nucleic acids are formed (heteroduplex and homoduplex nucleic acids are formed if the test nucleic acid has mutations, and only homoduplex nucleic acids are formed if the test nucleic acid does not have mutations).

The double-stranded nucleic acids can be denatured by exposing them to acidic or alkaline pH or to a high temperature in a solution. The pH can be changed by replacing the solution with, for example, 0.1 M NaOH or 0.1 M HCl. The temperature can be raised to the melting temperature (Tm) of the nucleic acid or higher. It is usually set to about 95°C.

Hybridization can be easily performed by returning the pH of the solution to neutral or lowering the temperature gradually to set it the same as or lower than Tm. For example, for a 200 base-pair nucleic acid, the same mole of a test nucleic acid and a control nucleic acid, or excess mole of the one to the other, is added in 6xSSC solution (90 mM sodium citrate (pH 7.2) and 0.9 M NaCl). The temperature is raised to 95°C once then gradually cooled to room temperature over 30 minutes to 2 hours. The mixture is then treated with MicroSpin S-300 HR column (Amersham Pharmacia Biotech) if the single-stranded nucleic acid that does not hybridize is removed.

Next, the double-stranded nucleic acid formed by hybridization is contacted with the MutM protein. The MutM protein used is the same as those described in the method of detecting mismatches mentioned above. The binding of the double-stranded nucleic acid to the MutM protein is then detected in the same manner as in detecting the binding of a test double-stranded nucleic acid to the MutM protein mentioned above.

Examples of the detection system are the same as mentioned above. However, when a nucleic acid is immobilized on or labeled so as to be immobilized on a support, either a test nucleic acid or a control nucleic acid can be immobilized or labeled.

If significant binding of a double-stranded nucleic acid formed by hybridization to MutM proteins is detected, it is judged that a test double-stranded nucleic acid has mismatches. In contrast, if significant binding of a double-stranded nucleic acid to MutM proteins is not detected, it is judged that a test double-stranded nucleic acid does not have mismatches.

In another aspect, the present invention relates to a method for separating a double-stranded nucleic acid with or without mismatches from a double-stranded nucleic acid sample using MutM. The principle of the method of the present invention is as follows. First, a double-stranded nucleic acid sample that is expected to contain a heteroduplex nucleic acid is prepared, and the MutM protein is contacted with this sample. Since the MutM protein binds only to a heteroduplex nucleic acid in the double-stranded nucleic acid sample, the heteroduplex nucleic acid binding to the MutM protein is collected if the MutM protein is collected from the double-stranded nucleic acid sample contacted with MutM. A homoduplex nucleic acid can be collected by removing the MutM protein and double-stranded nucleic acids binding to the protein from the double-stranded nucleic acid sample.

More specifically, the method of the present invention for separating a double-stranded nucleic acid with mismatches comprises
(a) contacting a double-stranded nucleic acid sample with the MutM protein and
(b) collecting a double-stranded nucleic acid that forms a complex with the MutM protein from the double-stranded nucleic acid sample.

The method of the present invention for separating a double-stranded nucleic acid without mismatches comprises
(a) contacting the double-stranded nucleic acid sample with the MutM protein and
(b) collecting a double-stranded nucleic acid that does not bind to the MutM protein from the double-stranded nucleic acid sample.

In the methods mentioned above, the purity can be increased, if necessary, by repeating processes (a) and (b) several times.

The method for detecting the cleavage of a test double-stranded nucleic acid by MutM is not limited. Examples of the detection system are as follows.
(1) A test double-stranded nucleic acid, which is labeled with fluorescent dye or radioisotope on one or both strands, is cleaved by MutM protein. The cleaved nucleic acid is subjected to electrophoresis through a matrix under denaturing conditions so that the individual strands separate according to their lengths, i.e. as in a DNA sequencer. If the amount of nucleic acid is sufficient, non-labeled nucleic acids can be used for electrophoresis and then detected by staining with silver or ethidium bromide.
(2) A test double-stranded nucleic acid is immobilized on a support or labeled so as to be immobilized on a support, and MutM protein is used to cleaving mismatches in the nucleic acid. Then the temperature of the sample is increased slowly to denature the double-stranded nucleic acid and cause the non-immobilized strand to elute. The melting temperature of short double-stranded nucleic acid such as a cleaved nucleic acid is lower than that of a longer, non-cleaved nucleic acid. In other words, cleaved strands of a double-stranded nucleic acid with mismatches elute first, while non-cleaved strands without mismatches elute later, effectively separating these nucleic acids. To increase the sensitivity, fluorescent substances such as FITC or radioactive substances such as ³⁵S or ³²P can be used. Alternatively, an organic solvent such as formamide is available to denature the double-stranded nucleic acid.

The method for separating the double-stranded nucleic acid without mismatches from a test double-stranded nucleic acid by MutM protein is not limited. An example of the separation method is as follows.

A test double-stranded nucleic acid is treated with MutM protein to introduce single nucleotide gaps into one or the other strand at mismatches, and then a nuclease such as S 1 nuclease is used to cleave the intact strand opposite such introduced gaps. Thus by the sequential action of these two enzymes, the sites of mismatches are rendered into complete double-stranded cleavages. Using the sequentially treated double-stranded nucleic acid as a template for polymerase chain reaction, only the non-cleaved sequence is amplified.

The method for modifying the double-stranded nucleic acid with mismatches by MutM protein is not limited. An example of the modifying method is as follows.

A double-stranded nucleic acid is treated with MutM protein. Ordinarily, the cleavage occurs on one strand at the mismatch. Then doing a nick translation reaction with DNA polymerase I using appropriately labeled nucleoside triphosphates, radioactive substances such as 32P, fluorescent substances such as Cy5 or affinity labels such as biotin can be incorporated into the double-stranded nucleic acid at and adjoining the sites initially occupied by mismatches.

These methods are useful for cloning various genes. The method for separating a double-stranded nucleic acid with mismatches can be used, for example, to collect single nucleotide polymorphism (SNP). Recently, human genome analysis has been making progress, and various SNPs have been collected all over the world to clarify the relationship between gene mutations and diseases. The method of the present invention is suitable for SNP collection because it enables collecting only mismatched nucleic acids. The method for separating a double-stranded nucleic acid with mismatches is also useful for cloning genes homologous with a certain gene. For example, when a homologue of a known gene derived from some organism is to be cloned from a different organism, this method makes it possible to select a gene whose sequence is similar to some extent but is partly different. Furthermore, a gene whose sequence is similar but not completely the same can be selectively cloned even from the same organism.

The method for separating a double-stranded nucleic acid without mismatches is useful for purifying DNA amplification products. When a gene is cloned, DNA fragments amplified by polymerase chain reaction (PCR) are often inserted into a cloning vector. In this case, mutations are sometimes introduced into PCR-amplified products by DNA polymerase. The method of the present invention enables removing the DNA amplification products with mutations.

More specifically, separation can be performed by, for example, reacting the MutM protein with a double-stranded nucleic acid in a solution, reacting the resulting reaction mixture with an anti-MutM antibody, contacting the reaction products with protein A or protein G bound to a support such as beads, precipitating the immune complex by centrifugation to separate the double-stranded nucleic acid with or without mismatches to the supernatant or precipitate, respectively, and recovering them. The double-stranded nucleic acid with mismatches can be purified from the precipitate by suspending the precipitate in TE buffer (10 mM Tris-HCl (pH 8.0), 1 mM EDTA), adding three volumes of 3 M guanidine hydrochloride to denature the MutM protein and liberate the double-stranded nucleic acid. After centrifugation, the supernatant is collected as the DNA solution. The DNA contained in the solution can be used for cloning.

Furthermore, the separation can be performed using MutM protein immobilized on or labeled so as to be immobilized on a support. There is no limitation on the labeling substance or the support to be used. The supports include liquid chromatography carriers such as HiTrap NHS-activated (Amersham Pharmacia Biotech), magnetic beads such as Dynabeads M-450 Uncoated or M450-Tosylated (Dynal), and sensing elements of every kind of sensor such as sensor chip CM5 (Biacore) of surface plasmon resonance sensor. The MutM protein can be bound physically to Dynabeads M-450 Uncoated and chemically to the other supports. In HiTrap NHS-activated, the carboxyl groups of the Sepharose carrier are esterified with N-hydroxysuccinimide (NHS). If, after the base is substituted with low-pH solution such as 1 mM HCl, the MutM protein solution is contacted with the carrier, stable amide bonds are formed between the amino acids of the MutM protein and the carrier. Therefore, when the MutM protein reacted with double-stranded nucleic acid is contacted with the carrier, only double-stranded nucleic acid with mismatches can be trapped by the carrier; the double-stranded nucleic acid without mismatches can be separated and collected. The double-stranded nucleic acid with mismatches bound to the MutM protein can be collected by denaturing the MutM protein with 3 M guanidine hydrochloride.

The invention will now be further described with reference to the following nonlimiting Examples.

### Example 1

Double-stranded DNAs forty base pairs in length were formed with and without C containing mismatches by annealing oligonucleotide No. 15 with individually with oligonucleotides No. 16, No. 17, No. 18 or No. 19. Their sequences were as follows:

No.15: tgctgagctaatcgac[?]tgtcaagtcatgcgatacgtaac,

No. 16: Cy5-gttacgtatcgcatgacttgacaagtcgattagctcagca,

No. 17: Cy5-gttacgtatcgcatgacttgacacgtcgattagctcagca,

No. 18: Cy5-gttacgtatcgcatgacttgacaggtcgattagctcagca,

No. 19: Cy5-gttacgtatcgcatgacttgacatgtcgattagctcagca

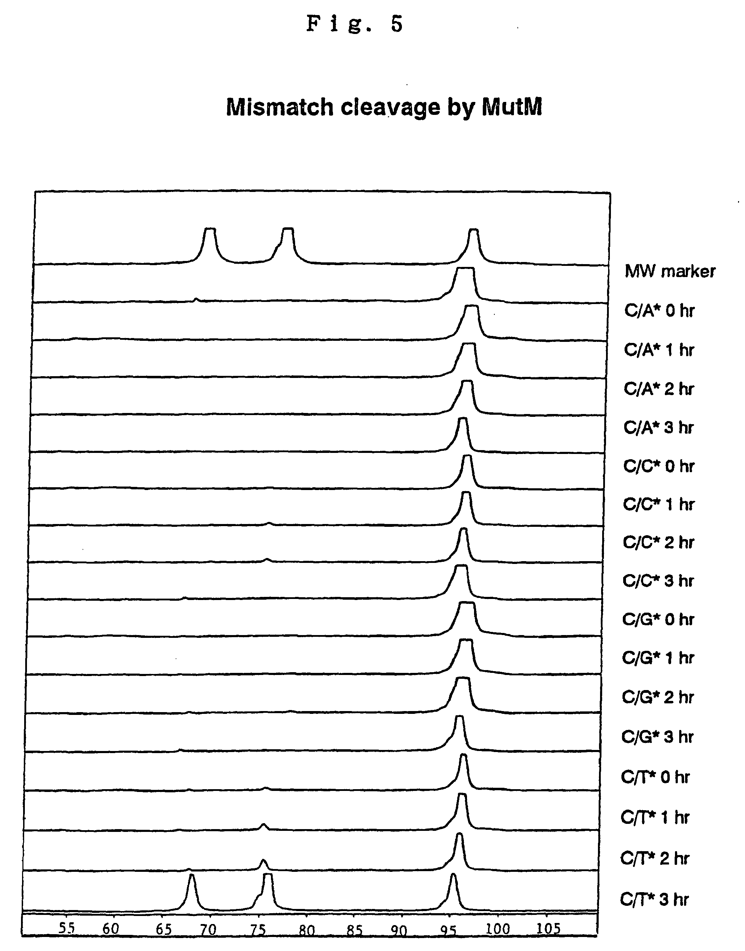

The double stranded DNAs were purified with MicroSpin S-200 HR columns (Amersham Pharmacia Biotech) and the concentrations of the purified DNAs were measured. Double-stranded DNAs (2―16 nM final concentration) and MutM protein (2 µM final concentration) were mixed in KCl buffer and incubated at 37° C for 1 to 3 hours. After the reaction, the cleavage activity was determined with an automated DNA sequencer by analysis of the DNA fragment length. Figure 5 shows results of the fragment analysis. It was found that C/C and C/T mismatches are substrates for cleavage by MutM.

### Example 2

The binding between a mismatched DNA and *E. coli* MutM was analyzed with an affinity sensor manufactured by BIACORE. The synthetic biotinylated oligonucleotide (B-gttggagcangtggtgttgg, SEQ ID NO: 1; B represents biotin, and n represents A, G, C, or T) was fixed on the sensor tip SA (BIACORE) at about 1,300 RU (1 RU corresponds to about 1 pg/mm² substance density) and annealed to the second synthetic oligonucleotide (ccaacaccacntgctccaac: SEQ ID NO: 2) in 6 x SSC (90 mM sodium citrate (pH 7.2), 0.9 M sodium chloride). In this process, about 1,200 RU oligonucleotide was annealed. The remaining single-stranded oligonucleotides were blocked by washing with 90 g/ml SSB (a single-stranded DNA binding protein). About 400 nM purified MutM was added, monitoring the binding to each double-stranded oligonucleotide. KCl buffer (50 mM Hepes-KOH (pH 7.2), 100 mM KCl, 1 mM EDTA, 1mM DTT, and 5 mM MgCl₂) was used as a running buffer. All manipulations were performed at 25°C.

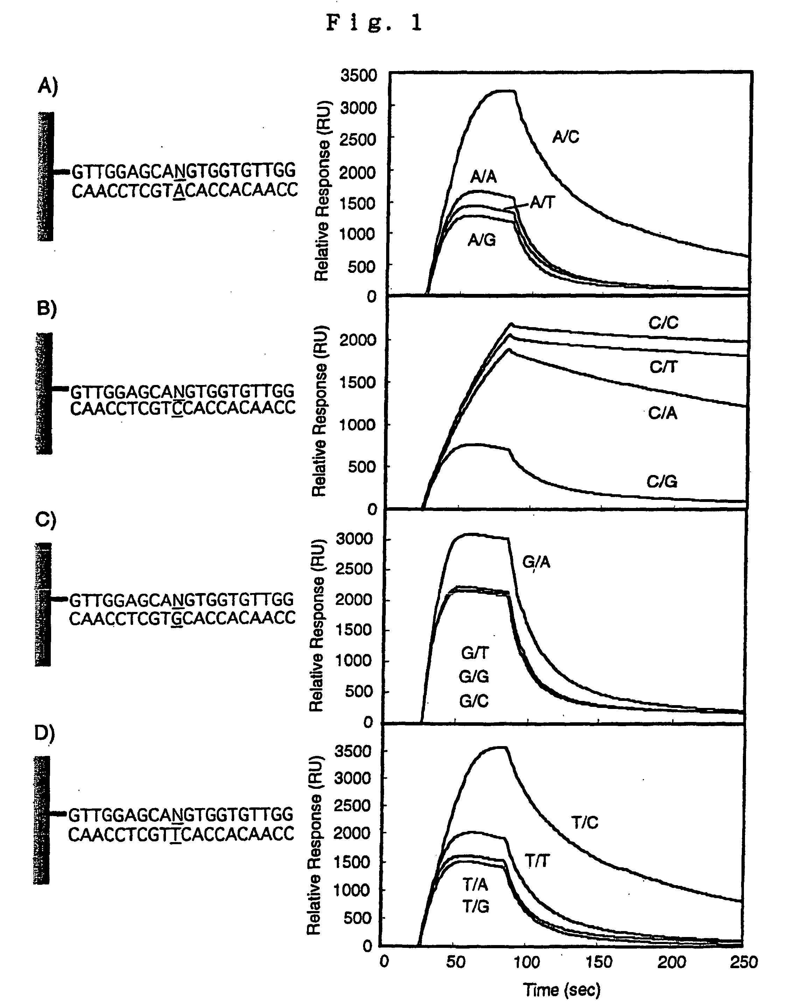

The result is shown in Fig. 1. A) shows interaction of MutM with the mismatches between A and A, C or G, and the A/T complementary double strands: B), interaction of MutM with the mismatches between C and A, C or T, and the C/G complementary double strands: C), interaction of MutM with the mismatches between G and A, G or T, and the G/C complementary double strands; and D), interaction of MutM with the mismatches between T and C, G or T, and the T/A complementary double strands. MutM was contacted with the double-stranded oligonucleotides for 25 sec to 1 min, and switched to KCl buffer from 85 sec to wash out non-specific absorbed substances. As is clear from Fig. 1, MutM was srongly bound to C/C, C/T and T/C, and C/A and A/C, while it was not bound to any complementary double-stranded oligonucleotides, such as A/T, C/G, G/C, and T/A.

### Example 3

*E. coli* MutM was used to purify DNA amplification products. Two kinds of PCR primers (GTAGTTGAAGAATTCCTGAATGAGCCATTTATC, SEQ ID NO: 3; the *Eco*RI restriction site is underlined, and AGCGCCTGCAGCGGGGTGAGTGAATCCGGAT, SEQ ID NO: 4; the *Pst*I restriction site is underlined) (25 pmol each) were added to the five PCR Beads (Amersham Pharmacia Biotech), and the total volume was made to 25 µl with sterilized water. One platinum loopful of *E. coli* was suspended in this solution and PCR was conducted with 30 cycles at an annealing temperature of 55°C. The DNA fragment of 870 bp was amplified in this reaction. After the reaction, 25 µl of TE buffer (10 mM Tris-HCl (pH 8.0), 1 mM EDTA) was added to the reaction mixture and the unreacted primers were removed using Microspin column (MicroSpin S-400 HR Amersham Pharmacia Biotech). Twenty-one microliters of 20 X SSC buffer were added to 50 µl of the purified PCR product solution, and annealing was performed by heating to 95°C and then gradually cooling. The unannealed single-stranded DNA was removed by the Microspin column, the same volume of 2 X KCl buffer was added to the reaction mixture, and the all the reaction mixtures in the five beads were gathered to pass through a MutM coupled affinity column described below.

One milliliter of 100 µM MutM solution was coupled to HiTrap NHS-activated column (Amersham Pharmacia Biotech) following the protocol. After blocking, the column was equilibrated with KCl buffer. The DNA solution was passed through the MutM coupled column. The fraction that passed through the colomun was collected and concentrated to 10 µl by ethanol precipitation. To the resulting concentrate were added 3 µl of OPA buffer accompanied with restriction enzymes (Amersham Pharmacia Biotech), 10 units of restriction enzyme *Eco*RI, and 10 units of *Pst*I. The total volume was made to 20 µl with sterilized water. The resulting reaction mixture was incubated at 37°C for 2 hours, mixed with 100 ng of *E coli* vector pTrc99A (Amersham Pharmacia Biotech) that were digested with *Eco*RI and *Pst*I, and precipitated with ethanol to obtain a final volume of 10 µl. The PCR fragment was ligated to the vector through the ligation reaction, and the ligation product was introduced into *E. coli* XL1-Blue. The DNA insert in the vector contained in 17 transformants obtained was amplified by colony PCR using the same PCR primers used in the first reaction.

The efficiency of mutagenesis in the DNA insert was evaluated by analyzing about 300 bp nucleotide sequence with a sequencing primer (Cy5-ACCGGTCCGAACATGGGCGGTAAA, SEQ ID NO: 5). As a result, the 17 clones that were subjected to the nucleotide sequence analysis did not contain any mutated DNA insert.

### Example 4

MutM was used to clone genes with mutation. One milliliter of 3M guanidine hydrochloride was passed through the MutM coupled column, to which the DNA was applied in Example 2, to recover the bound DNA. The collected DNA fraction was concentrated to 10 µl by ethanol precipitation and used to transform the DNA vector pTrc99A in the same manner as in Example 2. The nucleotide sequence analysis of the DNA insert in the vectors contained in three transformants obtained detected the mutation from G to C in one clone.

### Example 5

The binding between a DNA having continuous mismatches with different length and *E. coli* MutM was analyzed with an affinity sensor. The synthetic biotinylated oligonucleotides (B-tggtggttggagcaggtggtgttgggaaaa, SEQ ID NO: 6; B-tggtggttggagcacgtggtgttgggaaaa, SEQ ID NO: 7; B-tggtggttggagcaccgtggtgttgggaaaa, SEQ ID NO: 8; B-tggtggttggagcacccgtggtgttgggaaaa, SEQ ID NO: 9; and B-tggtggttggagcaccccgtggtgttgggaaaa, SEQ ID NO: 10; B represents biotin, and the mismatches are underlined) were fixed on the sensor tip SA (BIACORE) at about 700 RU and annealed to the second synthetic oligonucleotides (ttttcccaacaccacctgctccaaccacca, SEQ ID NO: 11 for SEQ ID NO: 6 and SEQ ID NO: 7, ttttcccaacaccaccctgctccaaccacca, SEQ ID NO: 12 for SEQ ID NO: 8, ttttcccaacaccacccctgctccaaccacca, SEQ ID NO. 13 for SEQ ID NO: 9, ttttcccaacaccaccccctgctccaaccacca, SEQ ID NO. 14 for SEQ ID NO: 10; the mismatches are underlined) were annealed in 6 x SSC. The running buffer was replaced by KCl buffer, and 200 nM purified MutM was applied to the column to monitor the binding to each double-stranded oligonucleotide in the same manner as in Example 1. All manipulations were conducted at 25°C.

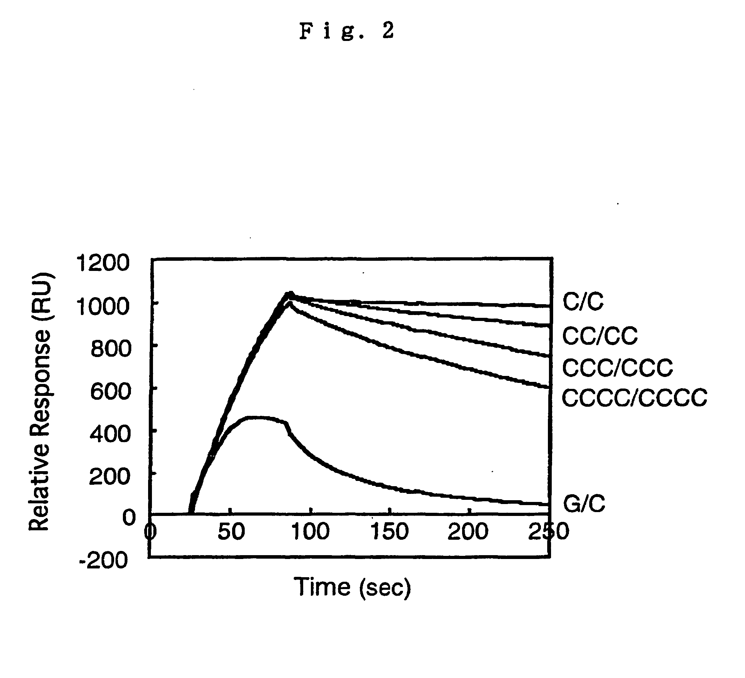

The result was shown in Fig. 2. The longer the continuous mismatch was, the less the amount of the bound MutM was. However, MutM had the obviously higher binding activity compared with the complementary double strands used as a control.

### Example 6

The binding between a DNA having C/C mismatches and several nucleotide bases of C in one strand, and *E. coli* MutM was analyzed with an affinity sensor. The synthetic biotinylated oligonucleotide (SEQ ID NO: 7) was fixed on the sensor tip SA at about 700 RU and annealed to SEQ ID NO: 11, SEQ ID NO: 12, SEQ ID NO: 13, or SEQ ID NO: 14 in the same condition as in Example 4, monitoring the binding of MutM to the double-stranded oligonucleotides in the same manner as in Example 4.

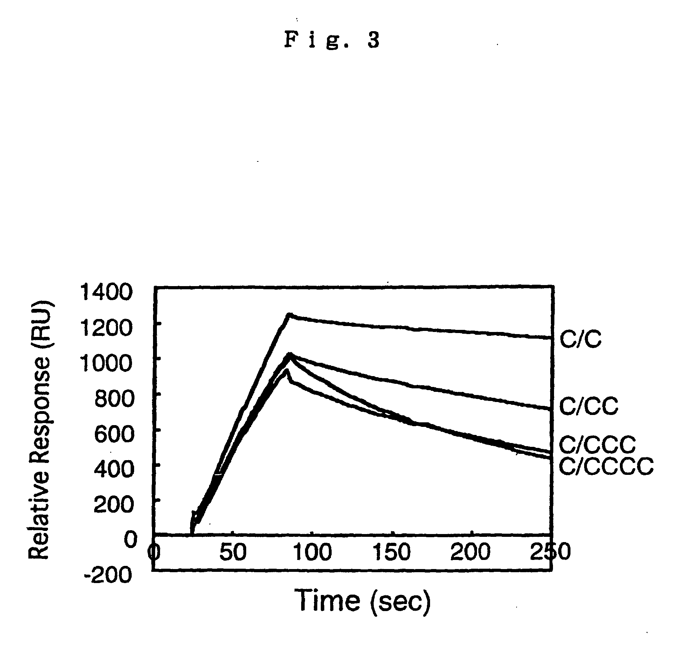

The result is shown in Fig. 3. The amount of the bound MutM decreased as the number of inserted C increased. However, the binding at 400 RU or higher was observed even at 250 sec, demonstrating that such mutation can be detected by MutM.

### Example 7

The binding between an insertion or deletion mutant DNA and *E. coli* MutM was analyzed with an affinity sensor. The synthetic biotinylated oligonucleotide (SEQ ID NO: 6) was fixed on the sensor tip SA at about 700 RU and annealed to SEQ ID NO: 11, SEQ ID NO: 12, SEQ ID NO: 13, or SEQ ID NO: 14 in the same condition as in Example 4, monitoring the binding of MutM to the double-stranded oligonucletides in the same manner as in Example 4.

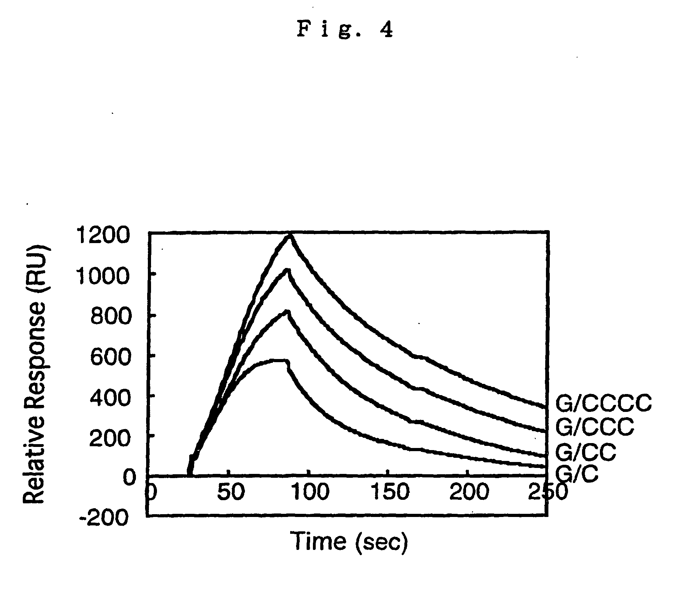

The result is shown in Fig. 4. The binding amount of MutM decreased as the number of the inserted nucleotide bases decreased. However, MutM exhibited higher binding activity than the control complementary DNA in any cases.

### Effect of the Invention

The present invention has revealed that MutM protein is capable of recognizing any mismatches involving cytosine in a nucleic acid. Efficient detection of mismatches in a double-stranded nucleic acid is enabled using this characteristic of the MutM protein. It has also become possible to efficiently separate a double-stranded DNA with mismatches from DNA without mismatches.

The methods of the present invention enable efficiently detecting mismatches between pyrimidines, which was difficult to be detected by the conventional method using MutS protein and separating a nucleic acid having such mismatches. Moreover, the present invention can be suitably applied to detection of multiple continuous mismatches, a mismatch between a single base and multiple bases, and further, the mismatches generated by deleting or inserting one or more bases in one strand of a double-stranded nucleic acid. The methods of the present invention are widely applicable to, for example, gene diagnosis or purification of DNA amplification products.

### Brief Description of the Invention

Figure 1 shows the oligonucleotide sequences used for evaluating the binding characteristic of MutM, and the results. In each panel, the oligonucleotide used was shown in the left, and the upper sequence in the double strand show the fixed oligonucleotide and the lower sequrence, the annealed sequence. "N" in the sequences represents any one of A, C, G or T. A) shows the binding to the double-stranded oligonucleotides having mismatches between A and N or complementary double-stranded oligonucleotides; B), the binding to the double-stranded oligonucleotides having mismatches between C and N or complementary double-stranded oligonucleotides; C), the binding to the double- stranded oligonucleotides having mismatches between G and N or complementary double-stranded oligonucleotides; and D), the binding to the double-stranded oligonucleotides having mismatches between T and N or complementary double-stranded oligonucleotides.

Figure 2 shows the result of analyzing the binding of the MutM protein to the DNA having continuous mismatches with different length, i.e., the DNA comprising 1 to 4 C/C continuous mismatches (C/C, CC/CC, CCC/CCC, and CCCC/CCCC), and to the DNA without mismatches (G/C).

Figure 3 shows the result of analyzing the binding between the MutM protein and the DNA with C/C mismatches and several bases of C at one strand.

Figure 4 shows the result of analyzing the binding between the insertion or deletion mutant DNA and the MutM protein.
Fig. 1
   Relative response
   Time (sec)
Fig. 2
   Relative response
   Time (sec)
Fig. 3
   Relative response
   Time (sec)
Fig. 4
   Relative response
   Time (sec)

## Claims

1. A method for detecting mismatches in a double-stranded nucleic acid, wherein the method comprises
(a) contacting a test double-stranded nucleic acid with MutM protein and
(b) detecting the binding of said double-stranded nucleic acid to said protein.

2. The method of claim 1, wherein MutM protein is derived from *E. coli*.

3. The method of claim 1 or 2, wherein the test double-stranded nucleic acid is binding to a support or labeled so as to bind to a support.

4. The method of claim 1 or 2, wherein MutM protein is binding to a support or labeled so as to bind to a support.

5. The method of any one of claims 1, 2, or 4, wherein the test double-stranded nucleic acid is detectably labeled.

6. The method of any one of claims 1 to 3, wherein MutM protein is detectably labeled.

7. A method for detecting mutations in a nucleic acid, wherein the method comprises
(a) providing a test nucleic acid and a control nucleic acid,
(b) hybridizing said test nucleic acid with the control nucleic acid,
(c) contacting the double-stranded nucleic acid formed by hybridization with MutM protein, and
(d) detecting the complex between heteroduplex nucleic acid in said double-stranded nucleic acid and said protein.

8. The method of claim 7, wherein MutM protein is derived from *E. coli.*

9. The method of claim 7 or 8, wherein the test nucleic acid or the control nucleic acid is binding to a support or labeled so as to bind to a support.

10. The method of claim 7 or 8, wherein MutM protein is binding to a support or labeled so as to bind to a support.

11. The method of any one of claims 7, 8, or 10, wherein the test nucleic acid or the control nucleic acid is detectably labeled.

12. The method of any one of claims 7 to 9, wherein MutM protein is detectably labeled.

13. A method for separating a double-stranded nucleic acid with mismatches from a double-stranded nucleic acid sample, wherein the method comprises
(a) contacting the double-stranded nucleic acid sample with MutM protein and
(b) collecting a double-stranded nucleic acid that forms a complex with MutM protein from the double-stranded nucleic acid sample.

14. A method for separating a double-stranded nucleic acid without mismatches from a double-stranded nucleic acid sample, wherein the method comprises
(a) contacting the double-stranded nucleic acid sample with MutM protein and
(b) collecting a double-stranded nucleic acid that does not bind to MutM protein from the double-stranded nucleic acid sample.

15. The method of claim 13 or 14, wherein MutM protein is derived from *E. coli*.

16. The method of any one of claims 13 to 15, wherein the double-stranded nucleic acid is a DNA amplification product.

17. The method of any one of claims 13 to 16, wherein MutM protein is binding to a support or labeled so as to bind to a support.

18. A method for detecting mismatches in a double stranded nucleic acid, wherein the method comprises
(a) contacting a test double-stranded nucleic acid with MutM protein and
(b) detecting the cleaving of said double-stranded nucleic acid by said protein.

19. A method for separating a double-stranded nucleic acid without mismatches from a double-stranded nucleic acid sample, wherein the method comprises
(a) contacting the double-stranded nucleic acid sample with MutM protein and
(b) collecting a double-stranded nucleic acid that is not cleaved by MutM protein from the double-stranded sample.

20. A method for modifying a mismatch-containing double-stranded nucleic acid, wherein the method comprises
(a) contacting the double-stranded nucleic acid sample with MutM protein and
(b) modifying further a double-stranded nucleic acid that is cleaved by MutM protein in the double-stranded sample.

## Patentansprüche

1. Verfahren zur Detektion von Fehlpaarungen in doppelsträngiger Nucleinsäure, wobei das Verfahren umfaßt
(a) Inkontaktbringen einer zu untersuchenden doppelsträngigen Nucleinsäure mit MutM-Protein und
(b) Detektieren der Bindung der doppelsträngigen Nucleinsäure an das Protein.

2. Verfahren nach Anspruch 1, worin MutM-Protein aus *E. coli* abgeleitet ist.

3. Verfahren nach Anspruch 1 oder 2, worin die zu untersuchende doppelsträngige Nucleinsäure an einen Träger bindet oder so markiert ist, daß sie an einen Träger bindet.

4. Verfahren nach Anspruch 1 oder 2, worin MutM-Protein an einen Träger bindet oder so markiert ist, daß es an einen Träger bindet.

5. Verfahren nach irgendeinem der Ansprüche 1, 2 oder 4, worin die zu untersuchende doppelsträngige Nucleinsäure detektierbar markiert ist.

6. Verfahren nach irgendeinem der Ansprüche 1 bis 3, worin das MutM-Protein detektierbar markiert ist.

7. Verfahren zur Detektion von Mutationen in Nucleinsäure, wobei das Verfahren umfaßt
(a) Vorsehen einer zu untersuchenden Nucleinsäure und einer Kontroll-Nucleinsäure,
(b) Hybridisieren der zu untersuchenden Nucleinsäure mit der Kontroll-Nucleinsäure,
(c) Inkontaktbringen der durch Hybridisierung gebildeten doppelsträngigen Nucleinsäure mit MutM-Protein, und
(d) Detektieren des Komplexes in der doppelsträngigen Nucleinsäure aus Heteroduplexnucleinsäure und Protein.

8. Verfahren nach Anspruch 7, worin das MutM-Protein aus *E. coli* abgeleitet ist.

9. Verfahren nach Anspruch 7 oder 8, worin die zu untersuchende Nucleinsäure oder die Kontroll-Nucleinsäure an einen Träger bindet oder so markiert ist. daß sie an einen Träger bindet.

10. Verfahren nach Anspruch 7 oder 8, worin das MutM-Protein an einen Träger bindet oder so markiert ist, daß es an einen Träger bindet.

11. Verfahren nach irgendeinem der Ansprüche 7, 8 oder 10, worin die zu untersuchende Nucleinsäure oder die Kontroll-Nucleinsäure detektierbar markiert ist.

12. Verfahren nach irgendeinem der Ansprüche 7 bis 9, worin das MutM-Protein detektierbar markiert ist.

13. Verfahren zur Trennung doppelsträngiger Nucleinsäure mit Fehlpaarungen aus einer doppelsträngigen Nucleinsäureprobe, wobei das Verfahren umfaßt
(a) Inkontaktbringen der doppelsträngigen Nucleinsäureprobe mit MutM-Protein und
(b) Abtrennen der doppelsträngigen Nucleinsäure, welche einen Komplex mit MutM-Protein bildet, aus der doppelsträngigen Nucleinsäureprobe.

14. Verfahren zur Trennung doppelsträngiger Nucleinsäure ohne Fehlpaarungen aus einer doppelsträngigen Nucleinsäureprobe, wobei das Verfahren umfaßt
(a) Inkontaktbringen der doppelsträngigen Nucleinsäureprobe mit MutM-Protein und
(b) Abtrennen der doppelsträngigen Nucleinsäure, welche nicht an das MutM-Protein bindet aus der doppelsträngigen Nucleinsäureprobe.

15. Verfahren nach Anspruch 13 oder 14, worin MutM-Protein aus *E. coli* abgeleitet ist.

16. Verfahren nach irgendeinem der Ansprüche 13 bis 15, worin die doppelsträngige Nucleinsäure ein DNA-Amplifikationsprodukt ist.

17. Verfahren nach irgendeinem der Ansprüche 13 bis 16, worin MutM-Protein an einen Träger bindet oder so markiert ist, daß es an einen Träger bindet.

18. Verfahren zur Detektion von Fehlpaarungen in einer doppelsträngigen Nucleinsäure, wobei das Verfahren umfaßt
(a) Inkontaktbringen einer zu untersuchenden doppelsträngigen Nucleinsäure mit MutM-Protein und
(b) Detektieren des Schneidens der doppelsträngigen Nucleinsäure durch das Protein.

19. Verfahren zur Trennung einer doppelsträngigen Nucleinsäure ohne Fehlpaarung aus einer doppelsträngigen Nucleinsäureprobe, wobei das Verfahren umfaßt
(a) Inkontaktbringen der doppelsträngigen Nucleinsäureprobe mit MutM-Protein und
(b) Abtrennen von doppelsträngiger Nucleinsäure, die nicht von MutM-Protein geschnitten wird, aus der doppelsträngigen Probe.

20. Verfahren zur Modifikation einer Fehlpaarung enthaltenden doppelsträngigen Nucleinsäure, wobei das Verfahren umfaßt
(a) Inkontaktbringen der doppelsträngigen Nucleinsäureprobe mit MutM-Protein und
(b) weiterhin Modifizieren einer doppelsträngigen Nucleinsäure, die von MutM-Protein in der doppelsträngigen Probe geschnitten wird.

## Revendications

1. Procédé pour détecter des mésappariements dans un acide nucléique bicaténaire, le procédé comprenant :
(a) le fait de mettre un acide nucléique bicaténaire à tester en contact avec une protéine MutM et
(b) le fait de détecter la liaison dudit acide nucléique bicaténaire à ladite protéine.

2. Procédé de la revendication 1, dans lequel la protéine MutM est dérivée d'*E*. *coli*.

3. Procédé de la revendication 1 ou 2, dans lequel l'acide nucléique bicaténaire à tester se lie à un support ou est marqué de façon à se lier à un support.

4. Procédé de la revendication 1 ou 2, dans lequel la protéine MutM se lie à un support ou est marquée de façon à se lier à un support.

5. Procédé de l'une quelconque des revendications 1, 2 ou 4, dans lequel l'acide nucléique bicaténaire à tester est marqué de façon détectable.

6. Procédé de l'une quelconque des revendications 1 à 3, dans lequel la protéine MutM est marquée de façon détectable.

7. Procédé pour détecter des mutations dans un acide nucléique, le procédé comprenant :
(a) le fait de fournir un acide nucléique à tester et un acide nucléique témoin ;
(b) le fait d'hybrider ledit acide nucléique à tester avec l'acide nucléique témoin,
(c) le fait de mettre l'acide nucléique bicaténaire formé par hybridation en contact avec une protéine MutM, et
(d) le fait de détecter le complexe entre l'acide nucléique hétéroduplexe dans ledit acide nucléique bicaténaire et ladite protéine.

8. Procédé de la revendication 7, dans lequel la protéine MutM est dérivée d'*E*. *coli*.

9. Procédé de la revendication 7 ou 8, dans lequel l'acide nucléique à tester ou l'acide nucléique témoin se lie à un support ou est marqué de façon à se lier à un support.

10. Procédé de la revendication 7 ou 8, dans lequel la protéine MutM se lie à un support ou est marquée de façon à se lier à un support.

11. Procédé de l'une quelconque des revendications 7, 8 ou 10, dans lequel l'acide nucléique à tester ou l'acide nucléique témoin est marqué de façon détectable.

12. Procédé de l'une quelconque des revendications 7 à 9, dans lequel la protéine MutM est marquée de façon détectable.

13. Procédé pour séparer un acide nucléique bicaténaire avec des mésappariements à partir d'un échantillon d'acide nucléique bicaténaire, le procédé comprenant :
(a) le fait de mettre l'échantillon d'acide nucléique bicaténaire en contact avec une protéine MutM et
(b) le fait de collecter un acide nucléique bicaténaire qui forme un complexe avec la protéine MutM à partir de l'échantillon d'acide nucléique bicaténaire.

14. Procédé pour séparer un acide nucléique bicaténaire sans mésappariement à partir d'un échantillon d'acide nucléique bicaténaire, le procédé comprenant :
(a) le fait de mettre l'échantillon d'acide nucléique bicaténaire en contact avec une protéine MutM et
(b) le fait de collecter un acide nucléique bicaténaire qui ne se lie pas à la protéine MutM à partir de l'échantillon d'acide nucléique bicaténaire.

15. Procédé de la revendication 13 ou 14, dans lequel la protéine MutM est dérivée d'*E. coli*.

16. Procédé de l'une quelconque des revendications 13 à 15, dans lequel l'acide nucléique bicaténaire est un produit d'amplification d'ADN.

17. Procédé de l'une quelconque des revendications 13 à 16, dans lequel la protéine MutM se lie à un support ou est marquée de façon à se lier à un support.

18. Procédé pour détecter des mésappariements dans un acide nucléique bicaténaire, le procédé comprenant
(a) le fait de mettre en contact un acide nucléique bicaténaire à tester avec une protéine MutM et
(b) le fait de détecter le clivage dudit acide nucléique bicaténaire par ladite protéine.

19. Procédé pour séparer un acide nucléique bicaténaire sans mésappariement à partir d'un échantillon d'acide nucléique bicaténaire, le procédé comprenant :
(a) le fait de mettre l'échantillon d'acide nucléique bicaténaire en contact avec une protéine MutM et
(b) le fait de collecter un acide nucléique bicaténaire qui n'est pas clivé par la protéine MutM à partir de l'échantillon bicaténaire.

20. Procédé pour modifier un acide nucléique bicaténaire contenant des mésappariements, le procédé comprenant :
(a) le fait de mettre l'échantillon d'acide nucléique bicaténaire en contact avec une protéine MutM et
(b) le fait de modifier ensuite un acide nucléique bicaténaire qui est clivé par la protéine MutM dans l'échantillon bicaténaire.
